# EUROPEAN PATENT SPECIFICATION

(11) **EP 2 564 894 B1**
(45) Date of publication and mention of the grant of the patent: **18.11.2015**
(21) Application number: 12183158.0
(22) Date of filing: 05.09.2012
(51) Int. Cl.: A61N 1/32, A61N 1/40, A61N 2/00, A61B 18/00, A61N 2/02, A61N 7/02

(54) **A device for cosmetic improvement of the skin**
Ästhetische Vorrichtung
Dispositif esthétique

(30) Priority: 05.09.2011 US 201161531029 P
(43) Date of publication of application: 06.03.2013
(73) Proprietor: Venus Concept Ltd, 21652 Karmiel (IL)
(72) Inventor: Ron Edoute, Oded, 69643 Tel Aviv (IL); Ron Edoute, Orit, 69643 Tel Aviv (IL)
(74) Representative: Caspary, Karsten

(56) References cited:
- WO-A1-03/079916
- WO-A2-2010/007614
- US-A1- 2004 267 252
- US-A1- 2007 239 075
- US-B1- 6 500 141

## Description

### FIELD OF THE INVENTION

This invention relates generally to means for providing cosmetic improvement to the skin. It specifically relates to means for providing cosmetic improvement to the skin that combine pulsed electromagnetic fields, diathermy, acoustic waves, vacuum, pressure applying means and pulsed electrical currents.

### BACKGROUND OF THE INVENTION

Improving the appearance of the skin has been the goal of many esthetic products and procedures for many years, since a tight skin, without wrinkles or cellulite, has a younger and more appealing appearance. Apart from age related changes, the skin also suffers from exposure to chemical and physical injuries, such as tobacco, cosmetics, esthetics and radiation from the sun and other sources. Those factors contribute to the decrease in collagen production, to reduced elasticity, and the appearance of wrinkles.

It is widely known that as the muscle deteriorates through the aging process, the attached skin loses it elasticity, causing the skin to sag and wrinkle. Strengthening relevant muscle groups restores and maintains the original shape and contour of the muscles. As the muscles get stronger, they get shorter and flatter, causing the attached skin to become firmer and smoothing wrinkles, thus improving the overall appearance. Additionally, since the blood supply to a contracting muscle is 10 times greater than of a muscle at rest, the additional supply of blood to the muscles being strengthened delivers vital oxygen and nutrients to the skin, revitalizing the tissue.

A few main approaches to tightening of the skin are common practice today. The surgical approach carries disadvantages related to the anesthesia, the surgical complications, and the healing process, which may cause scars. The chemical peel approach usually involves injury to the outermost layer of the skin - the epidermis - which may cause discoloration. Since collagen fibers are found in the dermis - the subcutaneous layer of the skin, and since heat was shown to contract these fibers and generate their production [Zelickson BD, Kist D, Bernstein E, Brown DB, Ksenzenko S, Bums J, Kilmer S, Mehregan D, Pope K. Histological and ultrastructural evaluation of the effects of a radiofrequency-based nonablative dermal remodeling device: a pilot study. Arch Dermatol. 2004 Feb;140(2):204-9], methods of differentially heating the dermis (deep tissue diathermy) have recently arisen.

A unique method of treating the dermis is called Pulsed Electromagnetic Fields (PEMF) therapy. This method usually employs electromagnetic radiation of different frequencies - ranging from static magnetic fields, through extremely low frequencies (ELF) to higher radiofrequencies (RF) - administered in pulses.

PCT Pat. Appl. No. PCT/IL2009/000644, published as document WO2010/007614, (henceforth **'644**), for example, discloses a device that uses a combination of pulses of electromagnetic energy, heating (diathermy), and electric current to improve the cosmetic appearance of the skin. In contrast to other devices known in the art, the device disclosed in **'644** relies on application of electrical currents of ~1 mA and voltages > 4 V in addition to heat and pulsed electromagnetic fields. It was found that these conditions provide better results than those provided by other devices known in the art.

Other methods known to have therapeutic effect is the use of ultrasound. Despite the advances made by the above, a need remains for a device that can provide cosmetic improvement to the skin in a more efficient, effective and long-lasting manner.

### SUMMARY OF THE INVENTION

The present invention is designed to meet this long-felt need. In particular, it discloses a device comprises a synergistic combination of skin treatments including acoustic, pulsed magnetic fields, and tissue diathermy.

The housing of the device is designed to be partially evacuated such that the skin is elevated into the housing, increasing the contact between the skin and the treatment probes.

The invention is defined by the device of independent claim 1. Preferred embodiments are defined by the dependent claims. Any disclosed methods using the device of the invention are merely exemplary.

It is another object of the present invention to provide the device as defined above, wherein said housing containing at least one port **400** through which air can enter and exit.

It is another object of the present invention to provide the device as defined above, additionally comprising at least one probe **200** made of an electrically and magnetically conductive material capable of transferring energy to the skin, said at least one probe attached to a surface of said housing such that when said device is in use, said probe is in physical contact with said skin.

It is another object of the present invention to provide the device as defined above, wherein said energy is heat being transferred to the skin;

It is another object of the present invention to provide the device as defined above, wherein said acoustic transducer **300** is made of a metallic material.

It is another object of the present invention to provide the device as defined above, additionally comprising at least one second coil **210** wrapped around at least a portion of said at least one probe.

It is another object of the present invention to provide the device as defined above, additionally comprising at least one second coil **210** being positioned within said probe.

It is another object of the present invention to provide the device as defined above, wherein said at least one first coil **310** wrapped around said at least a portion of said at least one probe.

It is another object of the present invention to provide the device as defined above, wherein said at least one acoustic transducer is a piezoelectric transducer.

It is another object of the present invention to provide the device as defined above, comprising a plurality of acoustic transducers, wherein a separate coil is wrapped around each acoustic transducer.

It is another object of the present invention to provide the device as defined above, comprising a plurality of acoustic transducers, wherein a single coil is wrapped around all of said acoustic transducers.

It is another object of the present invention to provide the device as defined above, comprising a plurality of probes.

It is another object of the present invention to provide the device as defined above, wherein each of said probes is characterized by substantially a different size and shape.

It is another object of the present invention to provide the device as defined above, wherein a single coil is wrapped around all of said acoustic transducers.

It is another object of the present invention to provide the device as defined above, wherein at least one coil is wrapped around at least one of said probes and at least one of said acoustic transducers.

It is another object of the present invention to provide the device as defined above, wherein said housing is adapted to produce, when the lip of said open end is placed against or in proximity to the skin and said ports are in fluid connection with means for evacuating the interior of said housing, a seal sufficiently air-tight that evacuation of air through said ports produces an at least partial vacuum within the interior of said housing.

It is another object of the present invention to provide the device as defined above, when the lip of said open end is placed against the skin and said ports are in fluid connection with means for evacuation of air through said ports produces an at least partial vacuum within the interior of said housing

It is another object of the present invention to provide the device as defined above, comprising a plurality of ports.

It is another object of the present invention to provide the device as defined above, wherein the diameter said housing is sufficiently large relative to its depth that when the lip of said open end is placed against or in proximity to the skin, said ports are in fluid connection with means for evacuating the interior of said housing upon evacuation, and the interior of said housing is evacuated, at least part of the skin enters said housing to a depth sufficient to make contact with said at least one port.

It is another object of the present invention to provide the device as defined above, wherein said means for evacuating the interior of said housing are adapted to apply either pulses of suction or a constant vacuum.

It is another object of the present invention to provide the device as defined above, further comprising:
means for exhausting air from said housing, said means in fluid connection with said ports;
a source of alternating electrical current in electrical connection with said transducers;
a first source of time-varying electric current in electrical connection with said first coil;
a source of electrical current in electrical connection with said at least one probe; and,
a source of heat in thermal contact with said at least one probe.

It is another object of the present invention to provide the device as defined above, additionally comprising an independent second source of time-varying electric current in electrical connection with said second coil.

It is another object of the present invention to provide the device as defined above, wherein said source of alternating electrical current is adapted to provide alternating electrical current that will drive said transducers such that said transducers produce acoustic (e.g., ultrasonic waves) of frequency between about 0.5Hz and about 40MHz and a power of between about 1 mW/cm² and about 100W/cm², more specifically between about 1 mW/cm² and about 10W/cm², a magnetic flux density from about 0 to about 3 Tesla, a magnetic field strength from about 0 to 50 Gauss, a pulse duration from 0.1 to 1000 msec.

It is another object of the present invention to provide the device as defined above, wherein said two sources of time-varying electric current are adapted to produce time-varying electrical currents of substantially the same frequency, amplitude, phase, and waveform.

It is another object of the present invention to provide the device as defined above, wherein said two sources of time-varying electric current are adapted to produce time-varying electrical currents in which at least one of the frequency, the amplitude, the phase, and the waveform differ.

It is another object of the present invention to provide the device as defined above, wherein the relative waveforms, amplitudes, phases and frequencies of the electric currents produced by said at least one sources of time-varying electric current are adapted to produce a predetermined pattern.

It is another object of the present invention to provide the device as defined above, wherein said source of time-varying electric current is adapted to produce a current that will induce in said at least one probe a time-varying magnetic field of a form chosen from the group consisting of square wave, sine wave, saw tooth wave, ramped wave, triangular pulses, and any mathematical combination thereof.

It is another object of the present invention to provide the device as defined above, wherein the peak magnetic field provided by said probes is between 0 and about 3T, specifically, 0 to 100 Gauss, more specifically 0 to 50 Gauss.

It is another object of the present invention to provide the device as defined above, wherein said time-varying magnetic field is in the form of pulses of duration of between about 0.1 milliseconds and about 1000 milliseconds.

It is another object of the present invention to provide the device as defined above, wherein said time-varying magnetic field has a frequency of between about 0.5 Hz and about 40 MHz.

It is another object of the present invention to provide the device as defined above, comprising a plurality of probes and further comprising at least one source of RF electrical current in electrical connection with said plurality of probes.

It is another object of the present invention to provide the device as defined above, wherein each of said probes is characterized by substantially a different size and shape.

It is another object of the present invention to provide the device as defined above, wherein each of said source of RF electrical current is adapted to provide to each of said plurality of probes with a substantially different RF frequency.

It is another object of the present invention to provide the device as defined above, wherein each of said plurality of probes is adapted to apply a substantially different RF frequency to the skin of said patient.

It is another object of the present invention to provide the device as defined above, further comprising temperature measurement means and control means adapted to regulate at least one said RF electrical current, said time-varying current, and said alternating current such that the measured skin temperature does not go outside of predetermined limits.

It is another object of the present invention to provide the device as defined above, wherein said predetermined limits are ambient temperature and 50 °C.

It is another object of the present invention to provide the device as defined above, wherein said predetermined limits are 30 °C and 100 °C.

It is another object of the present invention to provide the device as defined above, further comprising modulation means for modulating the output of at least one of the group consisting of said first source of time-varying current, said second source of time-varying current, and said source of alternating current.

It is another object of the present invention to provide the device as defined above, wherein said modulation means comprises means chosen from the group consisting of amplitude modulation, frequency modulation, phase modulation, and any combination of the above.

It is another object of the present invention to provide the device as defined above, wherein said modulation means comprises means chosen from the group consisting of pulse-width modulation, pulse-frequency modulation, pulse-code modulation (PCT), and pulse-density modulation.

It is another object of the present invention to provide the device as defined above, wherein said modulation means is adapted to provide ELF modulation of said current.

It is another object of the present invention to provide the device as defined above, further comprising means for cooling the skin.

It is another object of the present invention to provide the device as defined above, wherein said means for cooling the skin is chosen from the group consisting of a Peltier effect cooling device, irrigation with cool water, and means for blowing air.

It is another object of the present invention to provide a method for improving the cosmetic appearance of the skin, comprising:
obtaining a device comprising:
   a housing **100** comprising an open end adapted for being placed against the or in proximity to the skin;
   at least one acoustic transducer **300** adapted for producing ultrasonic waves, said acoustic transducer attached to a surface of said housing such that when said device is in use, said acoustic transducer is in physical contact with said skin; and,
   at least one first coil **310** wrapped around at least a portion of said at least one acoustic transducer;
placing said device such that said at least one acoustic transducer and at least one probe are in physical contact with or in proximity to the skin to be treated;
at least partially evacuating said housing until said skin is elevated sufficiently to make physical contact with said at least one port;
activating said source of alternating current, thereby activating said at least one acoustic transducer; and,
activating said first source of time-varying current, thereby producing an electric current in said first coil and a time-varying magnetic field in said at least one acoustic transducer.

It is another object of the present invention to provide the method as defined above, wherein said housing containing at least one port **400** through which air can enter and exit.

It is another object of the present invention to provide the method as defined above, additionally step of providing said device with at least one probe **200** made of an electrically and magnetically conductive material capable of transferring energy to the skin, said at least one probe attached to a surface of said housing such that when said device is in use, said probe is in physical contact with said skin.

It is another object of the present invention to provide the method as defined above, wherein each of said plurality of probes is adapted to apply a substantially different RF frequency to the skin of said patient.

It is another object of the present invention to provide the method as defined above, additionally step of providing additionally said device with at least one second coil **210** wrapped around at least a portion of said at least one probe.

It is another object of the present invention to provide the method as defined above, further comprising a step of activating a second source of time-varying current, thereby producing an electric current in said second coil and a time-varying magnetic field in said at least one probe.

It is another object of the present invention to provide the method as defined above, further comprising a step of providing said acoustic transducer **300** being made of a metallic material.

It is another object of the present invention to provide the method as defined above, additionally step of positioning said at least one second coil **210** within said probe.

It is another object of the present invention to provide the method as defined above, additionally step of disposing said at least one first coil **310** wrapped around said at least a portion of said at least one probe

It is another object of the present invention to provide the method as defined above, further comprising a step of maintaining for the duration of treatment the at least partial vacuum obtained in said step of evacuating said housing.

It is another object of the present invention to provide the method as defined above, wherein said step of activating said source of alternating current further comprises a step of activating said source of alternating current such that said at least one acoustic transducer produces ultrasonic waves of frequency between about 0.5 Hz and about 40M Hz and a power of between about 1 milli W/cm² and about 100W/cm², more specifically between about 1 milli W/cm² and about 10W/cm².

It is another object of the present invention to provide the method as defined above, wherein said step of activating said second source of time-varying current further comprises a step of activating said second source of time-varying current so as to produce a time-varying electric current of substantially the same frequency, amplitude, phase, and waveform as the current produced in said step of activating said first source of time-varying current.

It is another object of the present invention to provide the method as defined above, wherein said step of activating said second source of time-varying current further comprises a step of activating said second source of time-varying current so as to produce a time-varying electrical current in which at least one of the frequency, the amplitude, the phase, and the waveform differs from that produced in said step of activating said first source of time-varying current.

It is another object of the present invention to provide the method as defined above, wherein said step of activating said second source of time-varying current further comprises a step of activating said second source of time-varying current so as to produce a time-varying electrical current in which at least one of the frequency, the amplitude, the phase, and the waveform differs from that produced in said step of activating said first source of time-varying current so as to produce a predetermined beat pattern.

It is another object of the present invention to provide the method as defined above, wherein said step of activating said second source of time-varying current further comprises a step of activating said second source of time-varying current so as to induce in said at least one probe a time-varying magnetic field of a form chosen from the group consisting of square wave, sine wave, sawtooth wave, ramped wave, triangular pulses, and any mathematical combination thereof.

It is another object of the present invention to provide the method as defined above, wherein said step of activating said second source of time-varying current further comprises a step of activating said second source of time-varying current so as to induce in said at least one probe a time-varying magnetic field adapted to result in eddy currents in said skin.

It is another object of the present invention to provide the method as defined above, wherein said step of activating said second source of time-varying current further comprises a step of activating said second source of time-varying current so as to induce in said at least one probe a time-varying magnetic field with a peak of between 0 and about 3 T, specifically, 0 to 100 Gauss, more specifically 0 to 50 Gauss.

It is another object of the present invention to provide the method as defined above, wherein said step of activating said second source of time-varying current further comprises a step of activating said second source of time-varying current so as to induce in said at least one probe a time-varying magnetic field in the form of pulses of duration between about 0.1 milliseconds and about 1000 milliseconds.

It is another object of the present invention to provide the method as defined above, wherein said step of activating said second source of time-varying current further comprises a step of activating said second source of time-varying current so as to induce in said at least one probe a time-varying magnetic field with a frequency of between about 0.5 Hz and about 40 MHz.

It is another object of the present invention to provide the method as defined above, further comprising:
obtaining at least two probes;
placing said at least two probes in contact with said skin;
obtaining a source of RF electrical current;
placing said source of RF electrical current in electrical connection with at least two of said at least two probes; and,
activating said source of RF electrical current.

It is another object of the present invention to provide the method as defined above, further comprising:
obtaining temperature measurement means;
obtaining control means adapted to regulate at least one said RF electrical current, said time-varying current, and said alternating current; and,
controlling at least one of said RF electrical current, said time-varying current, and said alternating current such that the measured skin temperature does not go outside of predetermined limits.

It is another object of the present invention to provide the method as defined above, wherein said predetermined limits are ambient temperature and 50 °C.

It is another object of the present invention to provide the method as defined above, wherein said predetermined limits are 30 °C and 100 °C.

It is another object of the present invention to provide the method as defined above, further comprising a step of modulating the output of at least one of said first source of time-varying current, said second source of time-varying current, and said source of alternating current.

It is another object of the present invention to provide the method as defined above, wherein said step of modulating includes a step chosen from the group consisting of modulating the amplitude of said output, modulating the frequency of said output, modulating the phase of said output, and any combination of the above.

It is another object of the present invention to provide the method as defined above, wherein said step of modulating includes a step chosen from the group consisting of modulating the pulse width, modulating the pulse frequency, modulating the pulse code, and modulating the pulse density.

It is another object of the present invention to provide the method as defined above, further comprising a step of modulating said output at an Extremely Low Frequency (ELF).

It is another object of the present invention to provide the method as defined above, further comprising a step of cooling the skin.

It is another object of the present invention to provide the method as defined above, wherein said step of cooling the skin comprises a further step chosen from the group consisting of cooling the skin with a Peltier effect cooling device, irrigating the skin with cool water, and blowing air across the skin.

It is another object of the present invention to provide a device for providing cosmetic improvement to the skin (and any other dermis treatment), wherein said device comprises:
a housing **100** comprising an open end adapted for being placed against the or in proximity to the skin;
at least one acoustic transducer **300** adapted for producing ultrasonic waves, said acoustic transducer attached to a surface of said housing such that when said device is in use, said acoustic transducer is in physical contact with said skin; and,
at least one first coil **310** being positioned within at least a portion of said at least one acoustic transducer.

It is another object of the present invention to provide the device as defined above, wherein said housing containing at least one port **400** through which air can enter and exit.

It is another object of the present invention to provide the device as defined above, additionally comprising at least one probe **200** made of an electrically and magnetically conductive material capable of transferring energy to the skin, said at least one probe attached to a surface of said housing such that when said device is in use, said probe is in physical contact with said skin.

It is another object of the present invention to provide the device as defined above, wherein each of said probes is adapted to apply a substantially different RF frequency to the skin of said patient.

It is another object of the present invention to provide the device as defined above, wherein said energy is heat being transferred to the skin;

It is another object of the present invention to provide the device as defined above, wherein said acoustic transducer **300** is made of a metallic material.

It is another object of the present invention to provide the device as defined above, additionally comprising at least one second coil **210** wrapped around at least a portion of said at least one probe.

It is another object of the present invention to provide the device as defined above, additionally comprising at least one second coil **210** being positioned within said probe.

It is another object of the present invention to provide the device as defined above, wherein said at least one acoustic transducer is a piezoelectric transducer.

It is another object of the present invention to provide the device as defined above, comprising a plurality of acoustic transducers, wherein a separate coil is wrapped around each acoustic transducer.

It is another object of the present invention to provide the device as defined above, comprising a plurality of acoustic transducers, wherein a single coil is wrapped around all of said acoustic transducers.

It is another object of the present invention to provide the device as defined above, comprising a plurality of probes.

It is another object of the present invention to provide the device as defined above, wherein each of said probes is characterized by substantially a different size and shape.

It is another object of the present invention to provide the device as defined above, wherein a single coil is wrapped around all of said acoustic transducers.

It is another object of the present invention to provide the device as defined above, wherein at least one coil is wrapped around at least one of said probes and at least one of said acoustic transducers.

It is another object of the present invention to provide the device as defined above, wherein said housing is adapted to produce, when the lip of said open end is placed against or in proximity to the skin and said ports are in fluid connection with means for evacuating the interior of said housing, a seal sufficiently air-tight that evacuation of air through said ports produces an at least partial vacuum within the interior of said housing.

It is another object of the present invention to provide the device as defined above, wherein said housing is adapted to produce, when the lip of said open end is placed against the skin and said ports are in fluid connection with means for evacuation of air through said ports produces an at least partial vacuum within the interior of said housing.

It is another object of the present invention to provide the device as defined above, comprising a plurality of ports.

It is another object of the present invention to provide the device as defined above, wherein the diameter said housing is sufficiently large relative to its depth that when the lip of said open end is placed against or in proximity to the skin, said ports are in fluid connection with means for evacuating the interior of said housing upon evacuation, and the interior of said housing is evacuated, at least part of the skin enters said housing to a depth sufficient to make contact with said at least one port.

It is another object of the present invention to provide the device as defined above, wherein said means for evacuating the interior of said housing are adapted to apply either pulses of suction or a constant vacuum.

It is another object of the present invention to provide the device as defined above, further comprising:
means for exhausting air from said housing, said means in fluid connection with said ports;
a source of alternating electrical current in electrical connection with said transducers;
a first source of time-varying electric current in electrical connection with said first coil;
a source of electrical current in electrical connection with said at least one probe; and,
a source of heat in thermal contact with said at least one probe.

It is another object of the present invention to provide the device as defined above, additionally comprising an independent second source of time-varying electric current in electrical connection with said second coil.

It is another object of the present invention to provide the device as defined above, wherein said source of alternating electrical current is adapted to provide alternating electrical current that will drive said transducers such that said transducers produce ultrasonic waves of frequency between about 0.5Hz and about 40M Hz and a power of between about 1 milli W/cm² and about 100W/cm², more specifically between about 1 milli W/cm² and about 10W/cm².

It is another object of the present invention to provide the device as defined above, wherein said two sources of time-varying electric current are adapted to produce time-varying electrical currents of substantially the same frequency, amplitude, phase, and waveform.

It is another object of the present invention to provide the device as defined above, wherein said two sources of time-varying electric current are adapted to produce time-varying electrical currents in which at least one of the frequency, the amplitude, the phase, and the waveform differ.

It is another object of the present invention to provide the device as defined above, wherein the relative waveforms, amplitudes, phases and frequencies of the electric currents produced by said at least one sources of time-varying electric current are adapted to produce a predetermined beat pattern.

It is another object of the present invention to provide the device as defined above, wherein said source of time-varying electric current is adapted to produce a current that will induce in said at least one probe a time-varying magnetic field of a form chosen from the group consisting of square wave, sine wave, sawtooth wave, ramped wave, triangular pulses, and any mathematical combination thereof.

It is another object of the present invention to provide the device as defined above, wherein the peak magnetic field provided by said probes is between 0 and about 3 T, specifically, 0 to 100 Gauss, more specifically 0 to 50 Gauss.

It is another object of the present invention to provide the device as defined above, wherein said time-varying magnetic field is in the form of pulses of duration of between about 0.1 milliseconds and about 1000 milliseconds.

It is another object of the present invention to provide the device as defined above, wherein said time-varying magnetic field has a frequency of between about 0.5 Hz and about 40 MHz.

It is another object of the present invention to provide the device as defined above, comprising a plurality of probes and further comprising at least one source of RF electrical current in electrical connection with said plurality of probes.

It is another object of the present invention to provide the device as defined above, wherein each of said probes is characterized by substantially a different size and shape.

It is another object of the present invention to provide the device as defined above, wherein each of said source of RF electrical current is adapted to provide to each of said plurality of probes with a substantially different RF frequency.

It is another object of the present invention to provide the device as defined above, wherein each of said plurality of probes is adapted to apply a substantially different RF frequency to the skin of said patient.

It is another object of the present invention to provide the device as defined above, further comprising temperature measurement means and control means adapted to regulate at least one said RF electrical current, said time-varying current, and said alternating current such that the measured skin temperature does not go outside of predetermined limits.

It is another object of the present invention to provide the device as defined above, wherein said predetermined limits are ambient temperature and 50 °C.

It is another object of the present invention to provide the device as defined above, wherein said predetermined limits are 30 °C and 100 °C.

It is another object of the present invention to provide the device as defined above, further comprising modulation means for modulating the output of at least one of the group consisting of said first source of time-varying current, said second source of time-varying current, and said source of alternating current.

It is another object of the present invention to provide the device as defined above, wherein said modulation means comprises means chosen from the group consisting of amplitude modulation, frequency modulation, phase modulation, and any combination of the above.

It is another object of the present invention to provide the device as defined above, wherein said modulation means comprises means chosen from the group consisting of pulse-width modulation, pulse-frequency modulation, pulse-code modulation, and pulse-density modulation.

It is another object of the present invention to provide the device as defined above, wherein said modulation means is adapted to provide Extremely Low Frequency (ELF) modulation of said current.

It is another object of the present invention to provide the device as defined above, further comprising means for cooling the skin.

It is another object of the present invention to provide the device as defined above, wherein said means for cooling the skin is chosen from the group consisting of a Peltier effect cooling device, irrigation with cool water, and means for blowing air.

It is another object of the present invention to provide a method for improving the cosmetic appearance of the skin, comprising:
obtaining a device comprising:
   a housing **100** comprising an open end adapted for being placed against the or in proximity to the skin;
   at least one acoustic transducer **300** adapted for producing ultrasonic waves, said acoustic transducer attached to a surface of said housing such that when said device is in use, said acoustic transducer is in physical contact with said skin; and,
   at least one first coil **310** being positioned within at least a portion of said at least one acoustic transducer;
placing said device such that said at least one acoustic transducer and at least one probe are in physical contact with or in proximity to the skin to be treated;
at least partially evacuating said housing until said skin is elevated sufficiently to make physical contact with said at least one port;
activating said source of alternating current, thereby activating said at least one acoustic transducer; and,
activating said first source of time-varying current, thereby producing an electric current in said first coil and a time-varying magnetic field in said at least one acoustic transducer.

It is another object of the present invention to provide the method as defined above, additionally comprising step of providing said housing with at least one port **400** through which air can enter and exit.

It is another object of the present invention to provide the method as defined above, additionally step of providing said device with at least one probe **200** made of an electrically and magnetically conductive material capable of transferring energy to the skin, said at least one probe attached to a surface of said housing such that when said device is in use, said probe is in physical contact with said skin.

It is another object of the present invention to provide the method as defined above, additionally step of providing additionally said device with at least one second coil **210** wrapped around at least a portion of said at least one probe.

It is another object of the present invention to provide the method as defined above, further comprising a step of activating a second source of time-varying current, thereby producing an electric current in said second coil and a time-varying magnetic field in said at least one probe.

It is another object of the present invention to provide the method as defined above, further comprising a step of providing said acoustic transducer **300** being made of a metallic material.

It is another object of the present invention to provide the method as defined above, additionally step of positioning said at least one second coil **210** within said probe.

It is another object of the present invention to provide the method as defined above, additionally step of disposing said at least one first coil **310** wrapped around said at least a portion of said at least one probe

It is another object of the present invention to provide the method as defined above, further comprising a step of maintaining for the duration of treatment the at least partial vacuum obtained in said step of evacuating said housing.

It is another object of the present invention to provide the method as defined above, wherein said step of activating said source of alternating current further comprises a step of activating said source of alternating current such that said at least one acoustic transducer produces ultrasonic waves of frequency between about 0.5 Hz and about 40M Hz and a power of between about 1 milli W/cm² and about 100W/cm², more specifically between about 1 milli W/cm² and about 10W/cm².

It is another object of the present invention to provide the method as defined above, wherein said step of activating said second source of time-varying current further comprises a step of activating said second source of time-varying current so as to produce a time-varying electric current of substantially the same frequency, amplitude, phase, and waveform as the current produced in said step of activating said first source of time-varying current.

It is another object of the present invention to provide the method as defined above, wherein said step of activating said second source of time-varying current further comprises a step of activating said second source of time-varying current so as to produce a time-varying electrical current in which at least one of the frequency, the amplitude, the phase, and the waveform differs from that produced in said step of activating said first source of time-varying current.

It is another object of the present invention to provide the method as defined above, wherein said step of activating said second source of time-varying current further comprises a step of activating said second source of time-varying current so as to produce a time-varying electrical current in which at least one of the frequency, the amplitude, the phase, and the waveform differs from that produced in said step of activating said first source of time-varying current so as to produce a predetermined beat pattern.

It is another object of the present invention to provide the method as defined above, wherein said step of activating said second source of time-varying current further comprises a step of activating said second source of time-varying current so as to induce in said at least one probe a time-varying magnetic field of a form chosen from the group consisting of square wave, sine wave, sawtooth wave, ramped wave, triangular pulses, and any mathematical combination thereof.

It is another object of the present invention to provide the method as defined above, wherein said step of activating said second source of time-varying current further comprises a step of activating said second source of time-varying current so as to induce in said at least one probe a time-varying magnetic field adapted to result in eddy currents in said skin.

It is another object of the present invention to provide the method as defined above, wherein said step of activating said second source of time-varying current further comprises a step of activating said second source of time-varying current so as to induce in said at least one probe a time-varying magnetic field with a peak of between 0 and about 3 T, specifically, 0 to 100 Gauss, more specifically 0 to 50 Gauss.

It is another object of the present invention to provide the method as defined above, wherein said step of activating said second source of time-varying current further comprises a step of activating said second source of time-varying current so as to induce in said at least one probe a time-varying magnetic field in the form of pulses of duration between about 0.1 milliseconds and about 1000 milliseconds.

It is another object of the present invention to provide the method as defined above, wherein said step of activating said second source of time-varying current further comprises a step of activating said second source of time-varying current so as to induce in said at least one probe a time-varying magnetic field with a frequency of between about 0.5 Hz and about 40 MHz.

The It is another object of the present invention to provide the method as defined above, further comprising:
obtaining at least two probes;
placing said at least two probes in contact with said skin;
obtaining a source of RF electrical current;
placing said source of RF electrical current in electrical connection with at least two of said at least two probes; and,
activating said source of RF electrical current.

It is another object of the present invention to provide the method as defined above, further comprising:
obtaining temperature measurement means;
obtaining control means adapted to regulate at least one said RF electrical current, said time-varying current, and said alternating current; and,
controlling at least one of said RF electrical current, said time-varying current, and said alternating current such that the measured skin temperature does not go outside of predetermined limits.

It is another object of the present invention to provide the method as defined above, wherein said predetermined limits are ambient temperature and 50 °C.

It is another object of the present invention to provide the method as defined above, wherein said predetermined limits are 30 °C and 100 °C.

It is another object of the present invention to provide the method as defined above, further comprising a step of modulating the output of at least one of said first source of time-varying current, said second source of time-varying current, and said source of alternating current.

It is another object of the present invention to provide the method as defined above, wherein said step of modulating includes a step chosen from the group consisting of modulating the amplitude of said output, modulating the frequency of said output, modulating the phase of said output, and any combination of the above.

It is another object of the present invention to provide the method as defined above, wherein said step of modulating includes a step chosen from the group consisting of modulating the pulse width, modulating the pulse frequency, modulating the pulse code, and modulating the pulse density.

It is another object of the present invention to provide the method as defined above, further comprising a step of modulating said output at an Extremely Low Frequency (ELF).

It is another object of the present invention to provide the method as defined above, further comprising a step of cooling the skin.

It is lastly an object of the present invention to provide the method as defined above, wherein said step of cooling the skin comprises a further step chosen from the group consisting of cooling the skin with a Peltier effect cooling device, irrigating the skin with cool water, and blowing air across the skin.

It is another object of the present invention to provide a device for providing cosmetic improvement to the skin, wherein said device comprises:
a housing **100** comprising an open end adapted for being placed against the or in proximity to the skin;
at least one acoustic transducer **300** adapted for producing ultrasonic waves, said acoustic transducer attached to a surface of said housing such that when said device is in use, said acoustic transducer is in physical contact with said skin; and,
at least one first coil **310** in proximity to at least a portion of said at least one acoustic transducer.

It is another object of the present invention to provide the as defined above, wherein said housing comprising at least one port **400** through which air can enter and exit.

It is another object of the present invention to provide the as defined above, additionally comprising at least one probe **200** made of an electrically and magnetically conductive material capable of transferring energy to the skin, said at least one probe attached to a surface of said housing such that when said device is in use, said probe is in physical contact with said skin.

It is another object of the present invention to provide the as defined above, wherein each of said probes is adapted to apply a substantially different RF frequency to the skin of said patient.

It is another object of the present invention to provide the as defined above, wherein said energy is heat being transferred to the skin.

It is another object of the present invention to provide the as defined above, wherein said acoustic transducer **300** is made of a metallic material.

It is another object of the present invention to provide the as defined above, additionally comprising at least one second coil **210** wrapped around at least a portion of said at least one probe.

It is another object of the present invention to provide the as defined above, additionally comprising at least one second coil **210** being positioned within said probe.

It is another object of the present invention to provide the as defined above, wherein said at least one first coil **310** wrapped around said at least a portion of said at least one probe.

It is another object of the present invention to provide the as defined above, wherein said at least one acoustic transducer is a piezoelectric transducer.

It is another object of the present invention to provide the as defined above, additionally comprising a plurality of acoustic transducers, wherein a separate coil is wrapped around each acoustic transducer.

It is another object of the present invention to provide the as defined above, additionally comprising a plurality of acoustic transducers, wherein a single coil is wrapped around all of said acoustic transducers.

It is another object of the present invention to provide the as defined above, comprising a plurality of probes.

It is another object of the present invention to provide the as defined above, wherein each of said probes is characterized by substantially a different size and shape.

It is another object of the present invention to provide the as defined above, wherein a single coil is wrapped around all of said acoustic transducers.

It is another object of the present invention to provide the as defined above, wherein at least one coil is wrapped around at least one of said probes and at least one of said acoustic transducers.

### BRIEF DESCRIPTION OF THE DRAWINGS

The invention is now described with reference to the drawings, wherein
FIG. **1** presents schematic cross-sectional views of several preferred embodiments of the invention;
FIG. **2** presents a schematic illustration of a set of probes and coils according to one embodiment of the invention;
FIG. **3** presents a schematic illustration of the acoustic transducer and associated coil elements according to two embodiments of the invention; and,
FIGs. **4-5** present a schematic exterior 3-D view of one embodiment of the invention.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figure and described in the specification, but only as indicated in the accompanying claims, with the proper scope determined only by the broadest interpretation of said claims.

The present invention provides a device for providing cosmetic improvement to the skin (and any other dermis treatment), wherein said device comprises:
a housing **100** comprising an open end adapted for being placed against the or in proximity to the skin;
at least one RF probe **200** provided with a coil 210 for producing RF waves, said transducer attached to a surface of said housing such that when said device is in use, said RF transducer is in physical contact with said skin; and
at least one suction unit.

According to another embodiment of the present invention, the device further comprises at least one acoustic transducer **300.**

According to a further embodiment of the present invention, at least one first coil **310** is wrapped around at least a portion of said at least one acoustic transducer **300.**

According to another embodiment of the present invention, the housing, as any of the above, additionally comprising at least one port **400** through which air can enter and exit.

The term **"Eddy currents"** or **"Foucault currents"** refers hereinafter to currents induced in conductors, when a conductor is exposed to a changing magnetic field due to relative motion of the field source and conductor; or due to variations of the field with time.

The term **"Pulse-code modulation (PCM)"** refers hereinafter to a method used to digitally represent sampled analog signals.

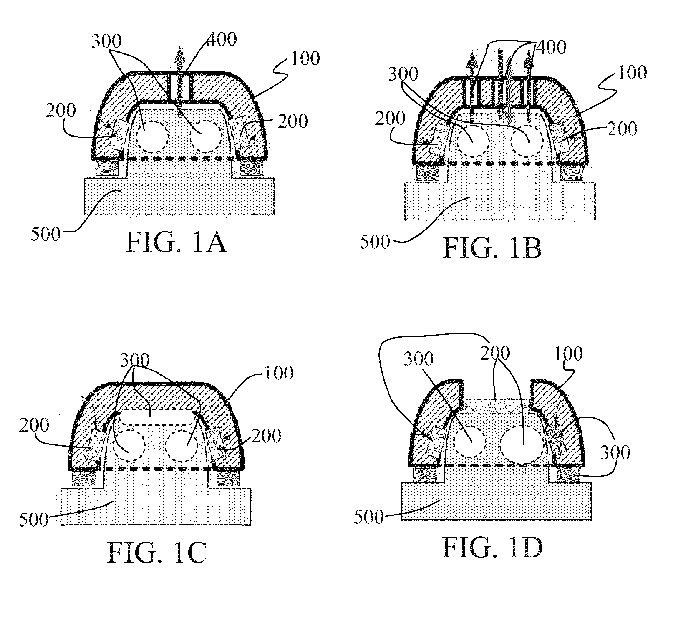

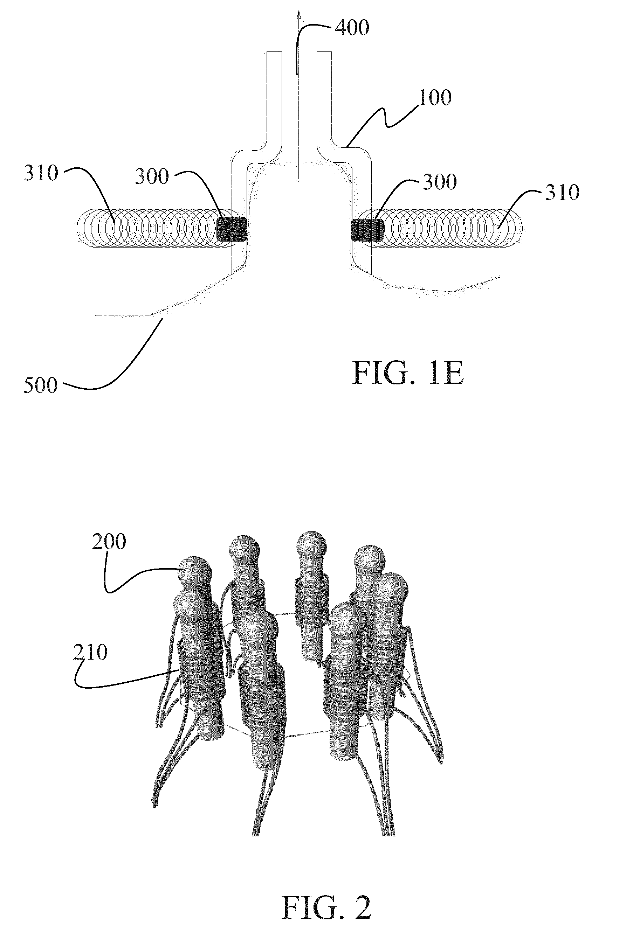

Reference is now made to FIG. **1****,** which presents schematic cross-sectional views of several preferred embodiments of the present invention. The device comprises a housing **100**, which presents a roughly cup-like shape, with an outer lip and a recessed inner region.

The substantially closed end of the housing comprises at least one port **400** which allows evacuation and introduction of air or other gas when the open end is covered, e.g. by the skin being treated. Disposed within the housing are at least one probe **200** (in the most preferred embodiments, a plurality of probes **200**), wrapped by coil **210** and at least one acoustic transducer **300** wrapped by coil **310**; coils **210** and **310** are not shown in FIG. 1.

The probes and transducers are described in detail below. As shown in FIG. **1**, the open end of the housing is placed on skin **500** to be treated. When the device is in use, as shown in the figure, suction is applied to the ports, causing the skin to be drawn into the housing.

In preferred embodiments, the skin is drawn in to a sufficient extent that it makes physical contact with the ports and any probes or transducers disposed about either the interior of the housing or the lip of the same.

According to another embodiment of the present invention said probes can apply either RF energy or Pulsed Electromagnetic Field Therapy (PEMF) or any combination thereof.

FIG. **1** provides schematic cross-sectional views of five exemplary embodiments of the device that provide non-limiting illustrations of possible geometries of the device. While these illustrations only show the elements that are disposed in the plane of the cross-section and a hidden view of a limited number of elements hidden by skin **500** that has been drawn into the housing, it is emphasized that the only limitations on the number of probes and acoustic transducers that can be used are the physical size of the housing and the minimum distance necessary to permit wrapping with the coil and to prevent short-circuiting.

FIG. **1A** shows an embodiment in which there is a single port **400;** the arrow indicates the direction of air flow when the device is in fluid connection with a source of vacuum. Probes **200** are disposed on the lip and interior surface of the housing, while acoustic transducers **300** are disposed on the interior surface of the housing. FIG. **1B** illustrates an embodiment in which the probes and transducers are disposed as shown in FIG. **1A**, but in which a plurality of ports **400** are provided. The arrows indicate the direction of air flow when the device is in fluid connection with a source of vacuum and when the air is let back in after the treatment has concluded.

FIG. **1C** shows an embodiment in which at least one transducer is disposed about the substantially closed end of the housing. FIG. **1D** shows an embodiment in which both acoustic transducers and probes are disposed about the lip of the housing and both acoustic transducers and probes are disposed about its inner surface. In this embodiment, at least one probe is disposed about the substantially closed end of the housing. In the embodiments illustrated in FIGs. **1C** and **1D**, the ports displaced from the central axis of the housing, and hence are not shown in the figures.

FIG. **IE** presents schematic illustration of an additional embodiment of the invention. In this embodiment, the housing has a generally cylindrical rather than cup-shaped housing, and the acoustic transducers are disposed about the interior surfaces of the sides of the housing, as shown in the figure.

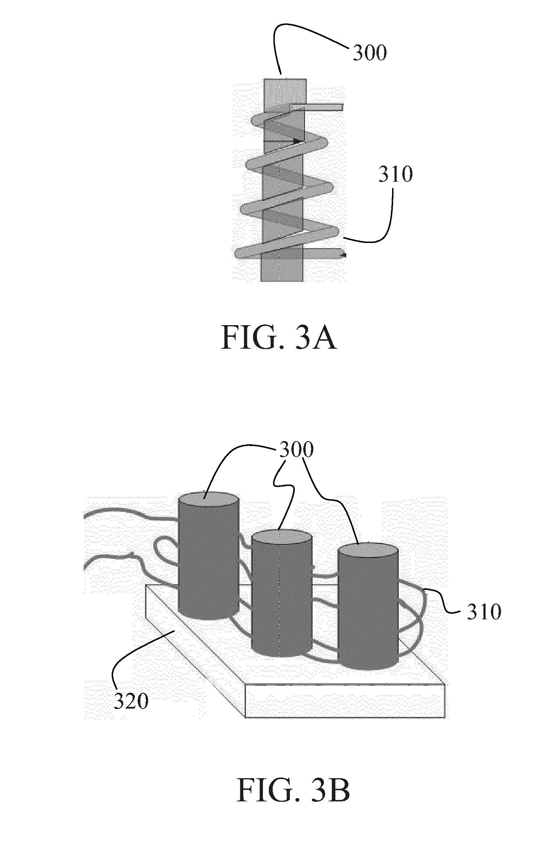

Reference is now made to FIG. **3**, which illustrates a plurality of probes **200** and coils **210** according to one embodiment of the invention. Probe **200** is made of a material (e.g. a piece of metal) capable of conducting electricity, magnetism, and heat. In preferred embodiments, it has a generally cylindrical shape. As used in the device herein disclosed, the probe is wrapped by a coil **210**. In the embodiment shown in the figure, each of the plurality of probes is wrapped by a coil made of a separate piece of wire. In other embodiments of the invention, not shown in the figure, coils **210** are constructed from a single piece of wire, i.e. the wire wraps one of the probes and the free end of the wire is then carried to the next probe, which it wraps to form a second coil, and so on. As will be appreciated by one skilled in the art, the coil can be made of any material that will carry a time-varying electrical current of the frequency and amplitude desired for the particular application (e.g. copper wire). When a time-varying electric current passes through the coil, a time-varying magnetic field is induced in the probe. In preferred embodiments, when the device is in use, the probes are in direct physical contact with or proximately to the skin to be treated, and the time-varying magnetic field is transmitted to the skin.

In preferred embodiments, the probes are also used for deep tissue diathermy. In these embodiments, at least two of the probes are placed in contact with the skin and act as electrodes. An RF electric current is passed between at least two probes via the skin, which is heated by the current passing therethrough. Techniques for deep tissue diathermy are well-known in the art. The probes can also be used to heat the skin directly by placing them in thermal contact with a heat source and with the skin to be treated.

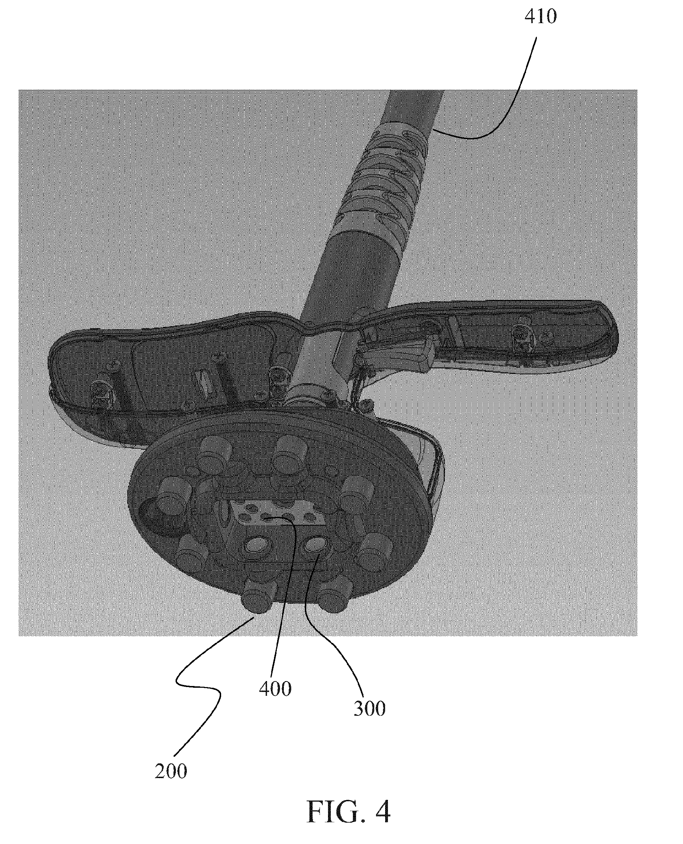

Reference is now made to FIG. **4**, which illustrates acoustic transducer **300** and coil **310** according to two embodiments of the invention. Acoustic transducer **300** may be any type of transducer known in the art for producing acustic. In preferred embodiments, the acoustic transducers are piezoelectric transducers, but other types (e.g. electromagnetic acoustic transducers) may be used as well; the device may comprise more than one type of transducer. The transducer is surrounded by a coil **310** capable of conducting a time-varying electric current. When a time-varying electric current passes through the coil, a magnetic field is created.

In preferred embodiments of the invention, when the device is in use, the acoustic transducers are in physical contact with the skin being treated.

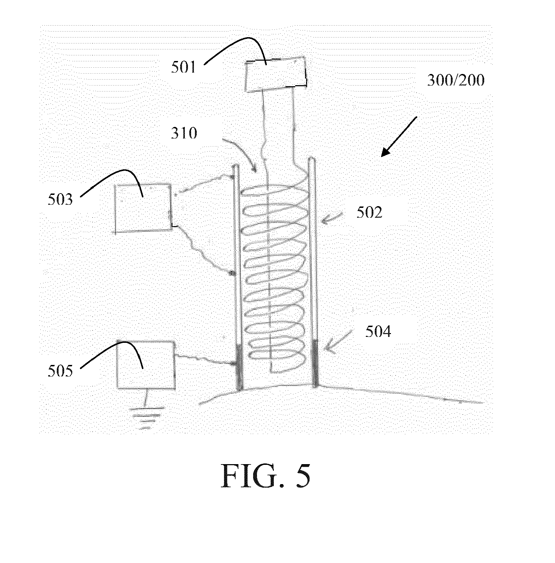

FIG. 3A illustrates a typical embodiment of a single acoustic transducer. The transducer **300** is wrapped with a coil **310**. As with the coils wrapping the probes, coil **310** may be made of any appropriate material capable of carrying a time-varying current of the frequency and amplitude desired. FIG. 3B illustrates a second embodiment comprising a plurality of transducers **300**. In this embodiment, the transducers are attached to a base **320** made of an appropriate non-conductive material such as plastic. Base **320** is attached to housing **100** such that the transducers are with the interior. Non-limiting examples of methods by which the base may be attached to the housing include gluing it to the interior surface; attaching it by attaching means such as screws; or cutting a hole through the wall of the housing (either slightly smaller than the base, or the same size as the base and leaving a lip so that the base cannot fall through) and attaching the base from the outside. In the embodiment illustrated in FIG. 3B, a single wire provides the coils for all of the plurality of the acoustic transducers. Reference is now made to FIG. **5**, which shows a schematic external 3-D view of the device. In the embodiment shown in the figure, the probes are disposed about the lip of the housing, while the acoustic transducers are disposed about the housing's inner surface. Also shown in the figure is the fluid connection **410** between the ports and an external source of vacuum.

In preferred embodiments of the invention, the device as described above further comprises appropriate external means for activating the components described above. In particular, in preferred embodiments of the invention, ports **400** are placed in fluid connection with means for creating an at least partial vacuum within the housing or suction on the ports. Any appropriate means for producing the partial vacuum known in the art (e.g. attachment to mechanical pumping means) may be used. When the device is in use, it is placed with the open end of the housing in contact with the skin to be treated, creating a seal around the lip of the housing. When the vacuum creation means are activated, the skin is drawn into the housing due to the partial vacuum.

In the some embodiments, the skin is drawn into the housing to a depth sufficient that physical contact is made with any probes or acoustic transducers disposed about the inner surface of the housing, and with the ports themselves.

In the some embodiments, the skin is drawn into the housing without being in physical contact with any probes or acoustic transducers disposed about the inner surface of the housing.

After the treatment is completed, the housing can be vented through the ports or by breaking the seal formed between the housing and the skin.

Further, in preferred embodiments, acoustic transducers **300** are placed in electrical contact with a source of AC current. Commercially available AC current sources may be used. Activation of the AC current source causes the transducers to create ultrasonic waves that are delivered to the skin to be treated. Acoustic therapy for skin rejuvenation is well-known in the art. In the most preferred embodiments of the invention, the frequency of the ultrasonic waves between about 0.5Hz and about 40M Hz and a power of between about 1 milli W/cm² and about 100W/cm², more specifically between about 1 milli W/cm² and about 10W/cm².

In preferred embodiments of the invention, coils **210** and **310** are placed in electrical contact with a source of time-varying electrical current. Commercially-available current sources (e.g. signal generators) may be used. The time-varying electrical current may be of any waveform desired and of any amplitude that will effect the desired cosmetic effects (it should be emphasized that the present invention can utilize the device of the present invention so as to provide any other dermis treatment); non-limiting examples include sinusoid (i.e. a typical AC signal), sawtooth, and square wave forms. In other embodiments, the time-varying electrical current is the form of pulses. The pulses may be of any shape; typical non-limiting waveforms include square pulses and triangular pulses. The time-varying electrical current passing through the coils induces a time-varying magnetic field that is then transmitted to the skin with which the coil or transducer is in contact.

These time-varying magnetic field provides a means for providing PEMF (pulsed electromagnetic field) therapy. There appear to be several mechanisms at work in PEMF. One mechanism appears to be purely thermal, that is, warming of the tissue treated due to absorption of the energy provided during the therapy. This thermal effect appears to result in contraction of collagen fibers, and skin rejuvenation.

Non-thermal effects also appear to be involved. These rely on the reactions of particular tissue components to the radiation applied to them. The specific mechanisms are poorly understood, but they appear to derive from the reaction of charged molecules both within and without the cells to the time-varying magnetic field. It has been observed that specific frequencies, duty cycles, and transmitted power are necessary to achieve a particular desired response in the tissue being treated.

Methods for producing a particular desired waveform are well-known in the art.

According to another embodiment, the time-varying current induce the time-varying magnetic field and results in eddy currents in the skin.

In other embodiments, the peak magnetic field can have any value up to 3 T, specifically, 0 to 100 Gauss, more specifically 0 to 50 Gauss. In preferred embodiments in which the time-varying magnetic field is in the form of pulses, the pulses have durations in the range of between about 0.1 ms and about 1000 ms. In other preferred embodiments, the time-varying magnetic field has a frequency of between about 0.5 Hz and about 1 MHz.

A single external source of current can be used to activate coils **210** and **310** simultaneously, in which case the current passing through the coils will perforce have the same waveform, frequency, amplitude, and phase. In other embodiments of the invention, separate external sources of current are used. In these embodiments, it is possible to produce two independent time-varying magnetic fields to be delivered to the skin being treated. It thus becomes possible, in some embodiments, to produce time-varying magnetic fields delivered from the two sets of coils in which the waveforms, frequencies, amplitudes, and/or phases differ. As will be appreciated by one skilled in the art, this effect is also possible even with a single current source by introduction of appropriate electronics into one or both of the circuits leading to the two sets of coils. It is known that in some cases, PEMF therapy is most effective when extremely low frequencies (ELFs) are used. By providing two independent time-varying magnetic fields with slightly differing frequencies, it is possible to provide ELF fields from the beats arising from the two signals. Thus, in some embodiments of the invention, the frequencies, amplitudes, phases, and waveforms of the two magnetic fields are chosen to provide a predetermined beat frequency and pattern. Means for calculating such patterns are well-known in the art.

In additional embodiments of the invention, means are provided for modulating the time-dependent signals, especially the RF currents used in the diathermy protocol. The modulation may be of any of the amplitude, frequency, or phase of the RF current, or any combination thereof. Non-limiting examples of modulation schemes that can be used in embodiments in which the time-varying signal is pulsed include pulse-width modulation, pulse-amplitude modulation, pulse-code modulation, and pulse-density modulation. As a non-limiting example, the current may be modulated at a very low frequency to provide an ELF time-varying current or magnetic field.

In preferred embodiments of the invention, in addition to ultrasonic treatment and PEMF therapy, the device is adapted for performing deep tissue diathermy. The general techniques of deep tissue diathermy are well-known in the art. In the embodiments that include means for performing deep tissue diathermy, the probes serve not only as electromagnets for the magnetic therapy, but also as electrodes. Embodiments in which the device is adapted for deep-tissue diathermy include at least two probes. These probes are connected to an external source of RF current and placed in physical contact with the skin to be treated. The RF current then flows subcutaneously, heating the tissue being treated.

In the most preferred embodiments of the invention, the diathermy unit is provided with safety mechanisms to prevent overheating of the tissue being treated. In these embodiments, the RF current source includes means for controlling the current delivered, and means for measuring the skin temperature are provided as well. Any appropriate means known in the art may be used. The amount of current is determined via a feedback loop to be sufficient to keep the skin temperature within predetermined limits, for example, between ambient temperature °C and 100 °C, more specifically ambient temperature and 50 °C. As the skin temperature increases, the current is cut back such that the rate of heating is lowered, while as the skin temperature decreases, the amount of current is increased so that more heat is generated in the tissue.

In additional embodiments of the device, it includes means for cooling the skin directly. These means may be any that are known in the art, e.g. a Peltier effect cooler in contact with the skin, irrigation with cool water, fanning with air, etc.

It is emphasized that the present invention provides a device that applies all of these treatment protocols (acoustic, PEMF, and deep tissue diathermy) *simultaneously* and with independent control. The present invention thus enables a synergistic combination of the three techniques, providing a unique convenient means for effecting cosmetic improvement of the skin that is more effective than use of any single technique. Furthermore, the effectiveness of the combination of the techniques provided by the current invention is greater than would have been expected from simply adding the known effects of each of the techniques when applied individually.

It is also within the scope of the invention to use the device as described above to provide a method for improving the cosmetic appearance of the skin. In a preferred embodiment of the method, any embodiment of the device (a non-limiting example of which is the embodiment illustrated in FIG. 5) is used. The device is connected to external sources of current and vacuum as described above. According to the method herein disclosed, the device is placed on the skin to be treated such that the acoustic transducer(s) and probe(s) are placed in physical contact with the skin (or such that they will be in contact once the housing is evacuated). The housing is then placed in fluid connection with a source of vacuum such that the skin is drawn into the housing and makes contact with the ports (and transducers and probes if it has not done so already). The current sources are then activated as described above, thereby providing simultaneously PEMF, acoustic, and diathermy. After the treatment, the current and vacuum sources are deactivated and the housing is vented.

It is also within the scope of the invention to disclose such a method, wherein the step of activating said source of alternating current further comprises a step of activating said source of alternating current such that said at least one acoustic transducer produces acoustic waves of frequency between about 0.5Hz and about 40M Hz and a power of between about 1 milli W/cm² and about 100W/cm², more specifically between about 1 milli W/cm² and about 10W/cm².

It is also within the scope of the invention to disclose such a method, wherein said step of activating said second source of time-varying current further comprises a step of activating said second source of time-varying current so as to produce a time-varying electric current of substantially the same frequency, amplitude, phase, and waveform as the current produced in said step of activating said first source of time-varying current.

It is also within the scope of the invention to disclose such a method, wherein said step of activating said second source of time-varying current further comprises a step of activating said second source of time-varying current so as to produce a time-varying electrical current in which at least one of the frequency, the amplitude, the phase, and the waveform differs from that produced in said step of activating said first source of time-varying current.

It is also within the scope of the invention to disclose such a method, wherein said step of activating said second source of time-varying current further comprises a step of activating said second source of time-varying current so as to produce a time-varying electrical current in which at least one of the frequency, the amplitude, the phase, and the waveform differs from that produced in said step of activating said first source of time-varying current so as to produce a predetermined beat pattern.

It is also within the scope of the invention to disclose such a method, wherein said step of activating said second source of time-varying current further comprises a step of activating said second source of time-varying current so as to induce in said at least one probe a time-varying magnetic field of a form chosen from the group consisting of square wave, sine wave, sawtooth wave, ramped wave, triangular pulses, and any mathematical combination thereof.

According to another embodiment, the time-varying current induce the time-varying magnetic field and results in eddy currents in the skin.It is also within the scope of the invention to disclose such a method, wherein said step of activating said second source of time-varying current further comprises a step of activating said second source of time-varying current so as to induce in said at least one probe a time-varying magnetic field with a peak of between 0 and about 3 T, specifically, 0 to 100 Gauss, more specifically 0 to 50 Gauss.

It is also within the scope of the invention to disclose such a method, wherein said step of activating said second source of time-varying current further comprises a step of activating said second source of time-varying current so as to induce in said at least one probe a time-varying magnetic field in the form of pulses of duration between about 0.1 milliseconds and about 1000 milliseconds.

It is also within the scope of the invention to disclose such a method, wherein said step of activating said second source of time-varying current further comprises a step of activating said second source of time-varying current so as to induce in said at least one probe a time-varying magnetic field with a frequency of between about 0.5 Hz and about 40 MHz.

It is also within the scope of the invention to disclose such a method, further comprising: obtaining temperature measurement means; obtaining control means adapted to regulate at least one said RF electrical current, said time-varying current, and said alternating current; and, controlling at least one of said RF electrical current, said time-varying current, and said alternating current such that the measured skin temperature does not go outside of predetermined limits.

It is also within the scope of the invention to disclose such a method, wherein said predetermined limits are ambient temperature and 50 °C.

It is also within the scope of the invention to disclose such a method, wherein said predetermined limits are 30 °C and 100 °C.

It is also within the scope of the invention to disclose such a method, further comprising a step of modulating the output of at least one of said first source of time-varying current, said second source of time-varying current, and said source of alternating current.

It is also within the scope of the invention to disclose such a method, further comprising a step of cooling the skin.

According to another embodiment of the present invention, either the acoustic transducer **300** or the probe **200** are made at partially hollow such that the coils are at least partially within said transducer **300** or the probe **200.**

Reference is now made to Fig. 5 which illustrates either a probe **200** or an acoustic transducer **300** in which the coil **310** is embedded within the same.

According to one embodiment, the coil **310** is coupled to a pulses generator **501.**

According to another embodiment, the acoustic transducer **300** is made of at least two sections: (i) a piezoelectric material, **502** (to which a pulses generator **503** is coupled); (b) a metallic part 504 which act as an electrode (to which a pulses generator **505** is coupled).

It is another object of the present invention to provide a device for providing cosmetic improvement to the skin, wherein said device comprises:
a housing **100** comprising an open end adapted for being placed against the or in proximity to the skin;
at least one acoustic transducer **300** adapted for producing ultrasonic waves, said acoustic transducer attached to a surface of said housing such that when said device is in use, said acoustic transducer is in physical contact with said skin; and,
at least one first coil **310** in proximity to at least a portion of said at least one acoustic transducer.

It is another object of the present invention to provide the as defined above, wherein said housing comprising at least one port **400** through which air can enter and exit.

It is another object of the present invention to provide the as defined above, additionally comprising at least one probe **200** made of an electrically and magnetically conductive material capable of transferring energy to the skin, said at least one probe attached to a surface of said housing such that when said device is in use, said probe is in physical contact with said skin.

It is another object of the present invention to provide the as defined above, wherein each of said probes is adapted to apply a substantially different RF frequency to the skin of said patient.

It is another object of the present invention to provide the as defined above, wherein said energy is heat being transferred to the skin.

It is another object of the present invention to provide the as defined above, wherein said acoustic transducer **300** is made of a metallic material.

It is another object of the present invention to provide the as defined above, additionally comprising at least one second coil **210** wrapped around at least a portion of said at least one probe.

It is another object of the present invention to provide the as defined above, additionally comprising at least one second coil **210** being positioned within said probe.

It is another object of the present invention to provide the as defined above, wherein said at least one first coil **310** wrapped around said at least a portion of said at least one probe.

It is another object of the present invention to provide the as defined above, wherein said at least one acoustic transducer is a piezoelectric transducer.

It is another object of the present invention to provide the as defined above, additionally comprising a plurality of acoustic transducers, wherein a separate coil is wrapped around each acoustic transducer.

It is another object of the present invention to provide the as defined above, additionally comprising a plurality of acoustic transducers, wherein a single coil is wrapped around all of said acoustic transducers.

It is another object of the present invention to provide the as defined above, comprising a plurality of probes.

It is another object of the present invention to provide the as defined above, wherein each of said probes is characterized by substantially a different size and shape.

It is another object of the present invention to provide the as defined above, wherein a single coil is wrapped around all of said acoustic transducers.

It is another object of the present invention to provide the as defined above, wherein at least one coil is wrapped around at least one of said probes and at least one of said acoustic transducers.

In the foregoing description, embodiments of the invention, including preferred embodiments, have been presented for the purpose of illustration and description. They are not intended to be exhaustive or to limit the invention to the precise form disclosed. Obvious modifications or variations are possible in light of the above teachings. The embodiments were chosen and described to provide the best illustration of the principals of the invention and its practical application, and to enable one of ordinary skill in the art to utilize the invention in various embodiments and with various modifications as are suited to the particular use contemplated. All such modifications and variations are within the scope of the invention as determined by the appended claims.

## Claims

1. A device for providing cosmetic improvement to the skin, wherein said device comprises:
a housing (100) comprising an open end adapted for being placed against the or in proximity to the skin;
at least one RF probe (200) provided with at least one first coil (210) for producing RF waves, said RF probe attached to a surface of said housing such that when said device is in use, said RF probe is in physical contact with said skin; at least one acoustic transducer (300);
at least one first coil (310) wrapped around at least a portion of said at least one acoustic transducer; and,
at least one suction unit.

2. The device according to claim 1, wherein said housing comprising at least one port (400) through which air can enter and exit.

3. The device according to claim 1, **characterized in that** said at least one RF probe (200) is made of an electrically and magnetically conductive material capable of transferring energy to the skin, said at least one probe attached to a surface of said housing such that when said device is in use, said probe is in physical contact with said skin.

4. The device according to claim 1, additionally comprising at least one second coil (210) wrapped around at least a portion of said at least one RF probe.

5. The device according to claim 1, additionally comprising at least one second coil (210) being positioned within said RF probe.

6. The device according to claim 5, **characterized in that** said at least one first coil (310) wrapped around said at least a portion of said at least one RF probe.

7. The device according to claim 1, comprising a plurality of acoustic transducers, wherein a separate coil is wrapped around each acoustic transducer.

8. The device according to claim 1, comprising a plurality of acoustic transducers, wherein a single coil is wrapped around all of said acoustic transducers.

9. The device according to claim 1, wherein said acoustic transducer produces acoustic waves of frequency between 5 MHz and 40 MHz.

10. The device according to claim 1, wherein the output power of said acoustic transducer is between 1 mW/cm² and 10 W/cm².

11. The device according to any one of claims 4-10, wherein at least one coil is wrapped around at least one of said probes and at least one of said acoustic transducers.

12. The device according to claim 1, **characterized in that** said housing is adapted to produce, when the lip of said open end is placed against or in proximity to the skin and said ports are in fluid connection with means for evacuating the interior of said housing, a seal sufficiently air-tight that evacuation of air through said ports produces an at least partial vacuum within the interior of said housing.

13. The device according to claim 1, **characterized in that** when a lip of said open end is placed against said skin and said ports are in fluid connection with means for evacuation of air through said ports, an at least partial vacuum is produced within the interior of said housing.

14. The device according to claim 1, **characterized in that** the diameter of said housing is sufficiently large relative to its depth that when a lip of said open end is placed against or in proximity to the skin, said ports are in fluid connection with means for evacuating the interior of said housing upon evacuation, and the interior of said housing is evacuated, at least part of the skin enters said housing to a depth sufficient to make contact with said at least one port.

15. The device according to either one of claims 13 or 14, **characterized in that** said means for evacuating the interior of said housing apply suction in a manner selected from the group consisting of pulses of suction and constant suction.

## Patentansprüche

1. Vorrichtung zur Bereitstellung von kosmetischer Hautverbesserung, wobei die Vorrichtung aufweist:
ein Gehäuse (100) mit einem offenen Ende, das geeignet ist, auf der Haut oder in der Nähe der Haut angeordnet zu werden;
mindestens eine Hochfrequenzsonde (200), die mit mindestens einer ersten Spule (210) zur Erzeugung von Hochfrequenzwellen versehen ist, wobei die Hochfrequenzsonde auf einer Oberfläche des Gehäuses befestigt ist, so dass, wenn die Vorrichtung in Verwendung ist, die Hochfrequenzsonde sich in physischem Kontakt mit der Haut befindet;
mindestens einen Schallwandler (300);
mindestens eine erste Spule (310), die um mindestens einen Abschnitt des mindestens einen Schallwandlers herum gewickelt ist; und
mindestens eine Saugeinheit.

2. Vorrichtung nach Anspruch 1, wobei das Gehäuse mindestens einen Anschluss (400) aufweist, durch den Luft eintreten und wieder austreten kann.

3. Vorrichtung nach Anspruch 1, **dadurch gekennzeichnet, dass** die mindestens eine Hochfrequenzsonde (200) aus einem elektrisch und magnetisch leitfähigen Material ausgebildet ist, das geeignet ist, Energie auf die Haut zu übertragen, wobei die mindestens eine Sonde auf einer Oberfläche des Gehäuses befestigt ist, so dass, wenn die Vorrichtung in Verwendung ist, die Sonde in physischem Kontakt mit der Haut ist.

4. Vorrichtung nach Anspruch 1, die zusätzlich mindestens eine zweite Spule (210) aufweist, die um mindestens einen Abschnitt der mindestens einen Hochfrequenzsonde herum gewickelt ist.

5. Vorrichtung nach Anspruch 1, die des Weiteren mindestens eine zweite Spule (210) aufweist, die innerhalb der Hochfrequenzsonde angeordnet ist.

6. Vorrichtung nach Anspruch 5, **dadurch gekennzeichnet, dass** die mindestens eine erste Spule (310) um mindestens einen Abschnitt der mindestens einen Hochfrequenzsonde herum gewickelt ist.

7. Vorrichtung nach Anspruch 1, die eine Mehrzahl von Schallwandlern aufweist, wobei eine separate Spule um jeden Schallwandler herum gewickelt ist.

8. Vorrichtung nach Anspruch 1, die eine Mehrzahl von Schallwandlern aufweist, wobei eine einzige Spule um sämtliche Schallwandler herum gewickelt ist.

9. Vorrichtung nach Anspruch 1, wobei der Schallwandler Schallwellen einer Frequenz zwischen 5 MHz und 40 MHz erzeugt.

10. Vorrichtung nach Anspruch 1, wobei die Ausgangsleistung des Schallwandlers zwischen 1 mW/cm² und 10 W/cm² beträgt.

11. Vorrichtung nach einem der Ansprüche 4 bis 10, wobei mindestens eine Spule um mindestens eine der Sonden und mindestens einen der Schallwandler herum gewickelt ist.

12. Vorrichtung nach Anspruch 1, **dadurch gekennzeichnet, dass**, wenn die Lippe des offenen Endes auf der Haut oder in der Nähe der Haut angeordnet ist, und die Anschlüsse in Fluidverbindung mit Mitteln zum Evakuieren des Inneren des Gehäuses sind, das Gehäuse geeignet ist, eine in ausreichendem Maße luftdichte Dichtung zu erzeugen, so dass eine Evakuierung von Luft durch die Anschlüsse mindestens ein Teilvakuum innerhalb des Inneren des Gehäuses erzeugt.

13. Vorrichtung nach Anspruch 1, **dadurch gekennzeichnet, dass**, wenn eine Lippe des offenen Endes auf der Haut angeordnet ist und wenn die Anschlüsse in Fluidverbindung mit Mitteln zur Evakuierung von Luft durch die Anschlüsse sind, mindestens ein Teilvakuum innerhalb des Inneren des Gehäuses erzeugt wird.

14. Vorrichtung nach Anspruch 1, **dadurch gekennzeichnet, dass** der Durchmesser des Gehäuses ausreichend groß in Bezug auf dessen Tiefe ist, so dass, wenn eine Lippe des offenen Endes auf der Haut oder in der Nähe der Haut angeordnet ist, die Anschlüsse in Fluidverbindung mit Mitteln zur Evakuierung des Inneren des Gehäuses bei Evakuierung sind und das Innere des Gehäuses evakuiert wird, mindestens ein Teil der Haut in das Gehäuse bis zu einer Tiefe eintritt, die ausreichend ist, um den Kontakt mit dem mindestens einen Anschluss herzustellen.

15. Vorrichtung nach einem der Ansprüche 13 oder 14, **dadurch gekennzeichnet, dass** die Mittel zum Evakuieren des Inneren des Gehäuses einen Sog in einer Art und Weise aufbringen, der aus der Gruppe ausgewählt ist, die aus Saugimpulsen und einen konstantem Sog besteht.

## Revendications

1. Dispositif pour fournir une amélioration cosmétique à la peau, dans lequel ledit dispositif comprend :
un boîtier (100) comprenant une extrémité ouverte adaptée pour être placée contre la peau ou à proximité de celle-ci ;
au moins une sonde RF (200) pourvue d'au moins une première bobine (210) pour produire des ondes RF, ladite sonde RF étant fixée à une surface dudit boîtier de telle sorte que lorsque ledit dispositif est en utilisation, ladite sonde RF soit en contact physique avec ladite peau ;
au moins un transducteur acoustique (300) ;
au moins une première bobine (310) étant enroulée autour d'au moins une portion dudit au moins un transducteur acoustique ; et,
au moins une unité de succion.

2. Dispositif selon la revendication 1, dans lequel ledit boîtier comprend au moins un orifice (400) à travers lequel de l'air peut entrer et sortir.

3. Dispositif selon la revendication 1, **caractérisé en ce que** ladite au moins une sonde RF (200) est faite d'un matériau électriquement et magnétiquement conducteur capable de transférer de l'énergie à la peau, ladite au moins une sonde étant fixée à une surface dudit boîtier de telle sorte que lorsque ledit dispositif est en utilisation, ladite sonde soit en contact physique avec ladite peau.

4. Dispositif selon la revendication 1, comprenant de plus au moins une seconde bobine (210) enroulée autour d'au moins une portion de ladite au moins une sonde RF.

5. Dispositif selon la revendication 1, comprenant de plus au moins une seconde bobine (210) étant positionnée à l'intérieur de ladite sonde RF.

6. Dispositif selon la revendication 5, **caractérisé en ce que** ladite au moins une première bobine (310) est enroulée autour de ladite au moins une portion de ladite au moins une sonde RF.

7. Dispositif selon la revendication 1, comprenant une pluralité de transducteurs acoustiques, dans lequel une bobine séparée est enroulée autour de chaque transducteur acoustique.

8. Dispositif selon la revendication 1, comprenant une pluralité de transducteurs acoustiques, dans lequel une seule bobine est enroulée autour de la totalité desdits transducteurs acoustiques.

9. Dispositif selon la revendication 1, dans lequel ledit transducteur acoustique produit des ondes acoustiques d'une fréquence entre 5 MHz et 40 MHz.

10. Dispositif selon la revendication 1, dans lequel la puissance de sortie dudit transducteur acoustique est entre 1 mW/cm² et 10 mW/cm².

11. Dispositif selon l'une quelconque des revendications 4 à 10, dans lequel au moins une bobine est enroulée autour d'au moins une desdites sondes et d'au moins un desdits transducteurs acoustiques.

12. Dispositif selon la revendication 1, **caractérisé en ce que** ledit boîtier est adapté pour produire, lorsque la lèvre de ladite extrémité ouverte est placée contre la peau ou à proximité de celle-ci et que lesdits orifices sont en liaison fluidique avec des moyens pour vidanger l'intérieur dudit boîtier, un joint suffisamment étanche à l'air pour que la vidange de l'air à travers lesdits orifices produise un vide au moins partiel à l'intérieur dudit boîtier.

13. Dispositif selon la revendication 1, **caractérisé en ce que** lorsqu'une lèvre de ladite extrémité ouverte est placée contre ladite peau et que lesdits orifices sont en liaison fluidique avec des moyens pour une vidange d'air à travers lesdits orifices, un vide au moins partiel est produit à l'intérieur dudit boîtier.

14. Dispositif selon la revendication 1, **caractérisé en ce que** le diamètre dudit boîtier est suffisamment grand par rapport à sa profondeur pour que lorsqu'une lèvre de ladite extrémité ouverte est placée contre la peau ou à proximité de celle-ci, que lesdits orifices sont en liaison fluidique avec des moyens pour vidanger l'intérieur dudit boîtier lors d'une vidange et que l'intérieur dudit boîtier est vidangé, au moins une partie de la peau pénètre dans ledit boîtier jusqu'à une profondeur suffisante pour faire contact avec ledit au moins un orifice.

15. Dispositif selon l'une ou l'autre des revendications 13 ou 14, **caractérisé en ce que** lesdits moyens pour vidanger l'intérieur dudit boîtier appliquent une succion d'une manière choisie parmi le groupe constitué d'impulsions de succion et d'une succion constante.
